(19) Europäisches Patentamt
European Patent Office
Office européen des brevets

(11) EP 3 122 317 B1

(12) # EUROPÄISCHE PATENTSCHRIFT

(45) Veröffentlichungstag und Bekanntmachung des Hinweises auf die Patenterteilung:
**02.10.2019 Patentblatt 2019/40**

(21) Anmeldenummer: **15701522.3**

(22) Anmeldetag: **27.01.2015**

(51) Int Cl.:
***A61K 8/35*** *(2006.01)* ***A61Q 17/04*** *(2006.01)*
***A61Q 19/00*** *(2006.01)* ***A61Q 19/08*** *(2006.01)*
***A61K 8/06*** *(2006.01)*

(86) Internationale Anmeldenummer:
**PCT/EP2015/051553**

(87) Internationale Veröffentlichungsnummer:
**WO 2015/144332 (01.10.2015 Gazette 2015/39)**

(54) **STABILE WASSER-IN-ÖL-EMULSIONEN MIT EINEM GEHALT AN 4-HYDROXYACETOPHENON**
STABLE WATER-IN-OIL EMULSIONS CONTAINING 4-HYDROXYACETOPHENONE
ÉMULSIONS EAU-DANS-HUILE STABLES CONTENANT DU 4-HYDROXYACÉTOPHÉNONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.03.2014 DE 102014104253**

(43) Veröffentlichungstag der Anmeldung:
**01.02.2017 Patentblatt 2017/05**

(73) Patentinhaber: **Symrise AG**
**37603 Holzminden (DE)**

(72) Erfinder:
- **PRUNS, Julia**
  **20144 Hamburg (DE)**
- **NISSEN, Bente**
  **20144 Hamburg (DE)**
- **RASCHKE, Thomas**
  **25421 Pinneberg (DE)**
- **VON WEDEL-PARLOW, Magdalena**
  **20251 Hamburg (DE)**
- **CHANTY, Delphine**
  **22767 Hamburg (DE)**

(74) Vertreter: **Fabry, Bernd**
**IP2 Patentanwalts GmbH**
**Schlossstrasse 523-525**
**41238 Mönchengladbach (DE)**

(56) Entgegenhaltungen:
**EP-A1- 2 774 481** **EP-A1- 2 774 604**
**WO-A1-03/070200** **WO-A2-2013/120829**
**JP-A- H07 206 645** **KR-A- 20130 134 976**

- **SYMRISE: "Multiple Benefits for Cosmetics with SymSave H", INTERNET CITATION, 26. Juli 2013 (2013-07-26), XP007923019, Gefunden im Internet: URL:http://www.symrise.com/newsroom/article/multiple-benefits-for-cosmetics-with-symsaveR-h/ [gefunden am 2015-02-25]**
- **RAJABI L ET AL: "Acetophenones with selective antimycobacterial activity", LETTERS IN APPLIED MICROBIOLOGY, OXFORD, GB , Bd. 40, Nr. 3 1. März 2005 (2005-03-01), Seiten 212-217, XP002693043, ISSN: 1472-765X, DOI: 10.1111/J.1472-765X.2005.01657.X Gefunden im Internet: URL:http://onlinelibrary.wiley.com/doi/10.1111/j.1472-765X.2005.01657.x/full [gefunden am 2013-02-18]**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 122 317 B1

**Beschreibung**

## GEBIET DER ERFINDUNG

Die vorliegende Erfindung betrifft Wasser-in-Öl-Emulsionen, welche gegen physikalische Zersetzung geschützt sind..

## TECHNOLOGISCHER HINTERGRUND

Die vorliegende Erfindung betrifft kosmetische bzw. dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Unter kosmetischer Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Bei alter Haut beispielsweise erfolgt die regenerative Erneuerung verlangsamt, wobei insbesondere das Wasserbindungsvermögen der Hornschicht nachlässt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen.

Bei pathologisch trockener und empfindlicher Haut liegt ein Barriereschaden a priori vor. Epidermale Interzellularlipide werden fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser. Die Barrierewirkung der Haut kann über die Bestimmung des transepidermalen Wasserverlustes (TEWL - transepidermal water loss) quantifiziert werden. Dabei handelt es sich um die Abdunstung von Wasser aus dem Körperinneren ohne Einbeziehung des Wasserverlustes beim Schwitzen. Die Bestimmung des TEWL-Wertes hat sich als außerordentlich informativ erwiesen und kann zur Diagnose rissiger oder schrundiger Haut, zur Bestimmung der Verträglichkeit chemisch verschiedenartig aufgebauter Tenside und dergleichen mehr herangezogen werden.

Für die Schönheit und Gepflegtheit der Haut ist der Wasseranteil in der obersten Hautschicht von größter Bedeutung. Man kann ihn in einem begrenzten Umfang durch Einbringen von Feuchtigkeitsregulatoren günstig beeinflussen.

Anionische Tenside, welche im allgemeinen Bestandteile von Reinigungszubereitungen sind, können den pH-Wert in der Hornschicht langanhaltend erhöhen, was regenerative Prozesse, die der Wiederherstellung und Erneuerung der Barrierefunktion der Haut dienen, stark behindert. In diesem Fall stellt sich in der Hornschicht zwischen Regeneration und dem Verlust essentieller Substanzen durch regelmäßige Extraktion ein neuer, häufig sehr ungünstiger Gleichgewichtszustand ein, der das äußere Erscheinungsbild der Haut und die physiologische Funktionsweise der Hornschicht entscheidend beeinträchtigt.

Schon bei einem einfachen Wasserbade ohne Zusatz von Tensiden kommt es zunächst zu einer Quellung der Hornschicht der Haut, wobei der Grad dieser Quellung beispielsweise von der Dauer des Bades und dessen Temperatur abhängt. Zugleich werden wasserlösliche Stoffe, z.B. wasserlösliche Schmutzbestandteile, aber auch hauteigene Stoffe, die für das Wasserbindungsvermögen der Hornschicht verantwortlich sind, ab- bzw. ausgewaschen. Durch hauteigene oberflächenaktive Stoffe werden zudem auch Hautfette in gewissem Ausmaße gelöst und ausgewaschen. Dies bedingt nach anfänglicher Quellung eine nachfolgende deutliche Austrocknung der Haut, die durch waschaktive Zusätze noch verstärkt werden kann.

Bei gesunder Haut sind diese Vorgänge im Allgemeinen belanglos, da die Schutzmechanismen der Haut solche leichten Störungen der oberen Hautschichten ohne weiteres kompensieren können. Aber bereits im Falle nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muss durch externe Maßnahmen regeneriert werden.

Darüber hinaus ist bekannt, dass Lipidzusammensetzung und -menge der Hornschicht der pathologisch veränderten, trockenen und der trockenen, jedoch nicht erkrankten Haut jüngerer und älterer Menschen vom Normalzustand abweicht, der in der gesunden, normal hydrierten Haut einer gleichalten Altersgruppe vorgefunden wird. Dabei stellen die Veränderungen im Lipidmuster der sehr trockenen, nicht-ekzematösen Haut von Patienten mit atopischem Ekzem einen Extremfall für die Abweichungen dar, die in der trockenen Haut hautgesunder Menschen vorgefunden werden.

Die Wirkung von Salben und Cremes auf Barrierefunktion und Hydratation der Hornschicht besteht in der Regel nicht in einer Wiederherstellung bzw. Stärkung der physikalischchemischen Eigenschaften der Lamellen aus Interzellularlipiden. Ein wesentlicher Teileffekt beruht auf der bloßen Abdeckung der behandelten Hautbezirke und dem daraus resultierenden Wasserstau in der darunterliegenden Hornschicht. Coapplizierte hygroskopische Substanzen binden das Wasser, so dass es zu einer messbaren Zunahme des Wassergehaltes in der Hornschicht kommt. Diese rein physikalische Barriere kann jedoch relativ leicht wieder entfernt werden. Nach dem Absetzen des Produktes kehrt die Haut dann sehr schnell wieder den Zustand vor Behandlungsbeginn zurück. Darüber hinaus kann die Hautpflegewirkung bei regelmäßiger Behandlung nachlassen, so dass schließlich sogar während der Behandlung der Status quo wieder erreicht wird. Bei bestimmten Produkten verschlechtert sich der Zustand der Haut nach Absetzen unter Umständen vorübergehend. Eine nachhaltige Produktwirkung wird in der Regel also nicht oder nur in einem eingeschränkten Masse erreicht.

Um die defizitäre Haut bei ihrer natürlichen Regeneration zu unterstützen und ihre physiologische Funktion zu stärken, werden topischen Präparaten in neuerer Zeit zunehmend Interzellularlipidmischungen zugesetzt, die von der Haut zum Wiederaufbau der natürlichen Barriere verwendet werden sollen. Allerdings handelt es sich bei diesen Lipiden, insbesondere aber den Ceramiden, um sehr teure Rohstoffe. Zudem ist ihre Wirkung meist sehr viel geringer als erhofft.

Produkte zur Pflege, Behandlung und Reinigung trockener und strapazierter Haut sind an sich bekannt. Allerdings ist ihr Beitrag zur Regeneration einer physiologisch intakten, hydratisierten und glatten Hornschicht umfangmäßig und zeitlich begrenzt.

Die Wirkung von Salben und Cremes auf die Barrierefunktion und die Hydratation der Hornschicht beruht im Wesentlichen auf der Abdeckung (Okklusion) der behandelten Hautbezirke. Die Salbe oder Creme stellt sozusagen eine (zweite) künstliche Barriere dar, die den Wasserverlust der Haut verhindern soll. Entsprechend leicht kann diese physikalische Barriere - beispielsweise mit Reinigungsmitteln - wieder entfernt werden, wodurch der ursprüngliche, beeinträchtigte Zustand wieder erreicht wird. Darüber hinaus kann die Hautpflegewirkung bei regelmäßiger Behandlung nachlassen. Nach dem Absetzen der Produktanwendung kehrt die Haut sehr schnell wieder in den Zustand vor Behandlungsbeginn zurück. Bei bestimmten Produkten verschlechtert sich der Zustand der Haut unter Umständen sogar vorübergehend. Eine nachhaltige Produktwirkung wird in der Regel also nicht oder nur in einem eingeschränkten Masse erreicht.

Die Wirkung einiger pharmazeutischer Zubereitungen auf die Barrierefunktion der Haut besteht sogar in einer selektiven Barriereschädigung, die ermöglichen soll, dass Wirkstoffe in bzw. durch die Haut in den Körper eindringen können. Ein gestörtes Erscheinungsbild der Haut wird dabei als Nebenwirkung teilweise billigend in Kauf genommen.

Die Wirkung von pflegenden Reinigungsprodukten besteht im Wesentlichen in einer effizienten Rückfettung mit Sebumlipid-ähnlichen Substanzen. Durch die gleichzeitige Verminderung des Tensidgehalts solcher Zubereitungen lässt sich der Schaden an der Hornschichtbarriere weiter begrenzen.

Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung der Hautalterung, insbesondere der durch oxidative Prozesse hervorgerufenen Hautalterung.

Antioxidantien sind Substanzen, welche Oxidationsprozesse verhindern bzw. welche die Autoxidation ungesättigte Verbindungen enthaltender Fette verhindern. Antioxidantien, welche auch auf dem Gebiete der Kosmetik und Pharmazie Verwendung finden sind beispielsweise a-Tocopherol, Sesamol, Gallensäurederivate, Butylhydroxyanisol und Butylhydroxytoluol.

Hauptsächlich werden Antioxidantien als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermato- logy", S. 323 und folgende (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

UV-Strahlung beispielsweise kann zu photooxidativen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxyradikale, Hydroperoxyradikale sowie Superoxidionen. Auch Undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls, kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Auch aus dem Grunde, solchen Reaktionen vorzubeugen, können kosmetischen bzw. dermatologischen Formulierungen zusätzlich Antioxidantien und/oder Radikalfänger einverleibt werden. Neben der UV-Strahlung induzieren

auch andere Umweltnoxen wie z.B. Ozon, Zigerattenrauch, oxidierende Chemikalien, Metallionen (Eisen, Nickel, Kupfer usw.) Schwefel- und Stickoxide oxidativen Stress in der Haut und leisten so vorzeitiger Hautalterung Vorschub.

Auch Hitzeeinwirkung kann zu einem beachtlichen Anstieg des Lipidperoxidspiegels der beeinträchtigten Haut führen, und sogar normale Stoffwechselvorgänge können oxidativen Stress induzieren.

## RELEVANTER STAND DER TECHNIK

Gegenstand der WO 2003 070200 A1 (Beiersdorf) offenbart kosmetische und/oder dermatologische Wasser-in-Öl-Emulsion enthaltend a) eine wässrige Phase mit einem Anteil von mehr als 70 Gew.-% bezogen auf die Gesamtmenge der Emulsion, b) einen W/O-Emulgator oder ein Gemisch aus mehreren W/O-Emulgatoren, gewählt aus der Gruppe der grenzflächenaktiven Substanzen der allgemeinen Struktur A-B-A', wobei A und A' gleiche oder verschiedenen hydrophobe organische Reste darstellen und B eine hydrophile Gruppe bedeutet, und/oder einen Silikonemulgator, in einer Konzentration von 1 bis 15 Gew.-%, c) sowie ein oder mehrere Tenside in einer Konzentration von 0,01 bis 10 Gew.-%, jeweils bezogen auf das Gesamtgewicht der Emulsion.

## AUFGABE DER ERFINDUNG

Ziel der vorliegenden Erfindung war es somit, Wege zu finden, die Nachteile des Standes der Technik zu vermeiden. Insbesondere sollte die Wirkung der Hautpflegeprodukte physiologisch, schnell und nachhaltig sein. Unter Hautpflege im Sinne der vorliegenden Erfindung ist in erster Linie zu verstehen, dass die natürliche Funktion der Haut als Barriere gegen Umwelteinflüsse (z. B. Schmutz, Chemikalien, Mikroorganismen) und gegen den Verlust von körpereigenen Stoffen (z. B. Wasser, Lipide, Elektrolyte) gestärkt oder wiederhergestellt wird.

Aufgabe der vorliegenden Erfindung war es weiterhin, den Nachteilen des Standes der Technik Abhilfe zu schaffen. Insbesondere war eine Aufgabe der vorliegenden Erfindung, Wirkstoffe und Produkte zur Verfügung zu stellen, welche in vivo als Antioxidantien wirken und/oder der durch oxidative Beanspruchung hervorgerufenen Hautalterung entgegenzuwirken.

Weiterhin war es eine Aufgabe der Erfindung, kosmetische Grundlagen für kosmetische Zubereitungen zu entwickeln, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen. Die Formulierung stabiler Wasser-in-Öl-Emulsionen mit einer überdurchschnittlichen Hautpflegeleistung (Hautbefeuchtung sowie Hautglättung) in Kombination mit sehr guten sensorischen Eigenschaften gestaltet sich derzeit als nach wie vor sehr schwierig. Dies ist nicht zuletzt darin begründet, dass der Zusatz hautpflegewirksamer Additive - wie z.B. Moisturizer, Antioxidantien, Konservierungsstoffe, Pigmente, bestimmte Lipide, Lösungsmittel oder Filmbildner - zu instabilen Emulsionen und/oder einem stumpfen bzw. klebrigen oder auch schmierigen Hautgefühl beim Verteilen des Produktes auf der Haut führt, so dass die Emulsionen dann zwar möglicherweise wirksam, aber gleichzeitig sensorisch unakzeptabel und/oder instabil sind.

Eine Aufgabe der vorliegenden Erfindung war es ferner, Grundlagen für Zubereitungen zur Verfügung zu stellen, welche es einesteils erlauben, höherviskose Zubereitungen zu erstellen (welche man landläufig als Cremes bezeichnen würde), aber auch andererseits eine gute Grundlage für Zubereitungen darstellen, die eine geringe oder gar sehr geringe Viskosität aufweisen (also beispielsweise Formulierungen, welche man landläufig als Lotionen bezeichnen würde), ohne aber von einigen der Nachteile des Standes der Technik behaftet zu sein.

Ganz besonders nachteilig an vielen Wasser-in-Öl-Emulsionen des Standes der Technik ist ferner, dass sie nicht stabil gegen physikalische Zersetzung sind. Üblicherweise bezeichnet man das am häufigsten auftretende unerwünschte Verhalten als "Ausölen". Dies besagt, dass sich Wasserphase und Ölphase allmählich trennen. Dies äußert sich beispielsweise darin, dass aus einer W/O-Creme (= Wasser-in-Öl-Creme) öltropfen austreten. Beginnt aber eine Emulsion erst einmal, sich zu zersetzen, so ist dieser Vorgang nicht mit einfachen Mitteln wieder rückgängig zu machen. Zumindest stehen dem Verbraucher solche Mittel nicht zur Verfügung.

Aufgabe der vorliegenden Erfindung war es also auch, die Nachteile des Standes der Technik in dieser Hinsicht zu beseitigen. Insbesondere sollten stabile hautpflegende kosmetische Mittel zur Verfügung gestellt werden.

## BESCHREIBUNG DER ERFINDUNG

Gegenstand der Erfindung sind W/O-Emulsionen enthaltend

(a) mindestens einen Silikonemulgator vom Typ der Alkyl-Dimethicon-Copolyole und

(b) 4-Hydroxyacetophenon.

## 4-HYDROXYACETOPHENON

Ein bekanntes und hochwirksames Antioxidans ist das 4-Hydroxyacetophenon, welches unter anderem von der Gesellschaft Symrise unter der Handelsbezeichnung "SymSave(R) H" verkauft wird. Es hat die CAS-Nr. 99-93-4 und zeichnet sich durch folgende chemische Struktur aus:

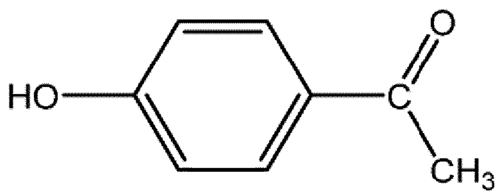

Der Stoff wird von der Firma Symrise AG unter der Bezeichnung SymSave H vertrieben, wird jedoch in der Firmenveröffentlichung *"Triple benefits for cosmetics with SymSave H"* nur im Zusammenhang mit Konservierung und Emulsionsstabilität beworben.

## EMULSIONEN

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

Unter Emulsionen versteht man im Allgemeinen heterogene Systeme, die aus zwei nicht o- der nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl- Emulsion (W/O-Emulsion, z. B. Butter) handelt es sich um das umgekehrte Prinzip, wobei der Grundcharakter hier durch das Öl bestimmt wird.

Es hat sich in der Vergangenheit durchaus bewährt, zumindest Teile einer kosmetisch verwendeten Ölphase aus der Gruppe der cyclischen und/oder linearen Silicone zu wählen, welche im Rahmen der vorliegenden Offenbarung auch als "Siliconöle" bezeichnet werden. Silicone haben jedoch einige Nachteile, hauptsächlich formulierungstechnischer Natur. Gleichwohl galten sie bisher als unverzichtbar, wollte man kosmetische Zubereitungen auf der Grundlage von Siliconölen formulieren.

Natürlich ist dem Fachmann eine Vielzahl von Möglichkeiten bekannt, stabile W/O-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind. Der Stand der Technik kennt allerdings nur wenige Formulierungen, die so dünnflüssig sind, dass sie beispielsweise sprühbar wären. Insbesondere Zubereitungen zur kosmetischen oder therapeutischen Hautpflege enthalten als wesentliche Bestandteile Abmischungen aus ölen bzw. Öllöslichen Substanzen und Wasser bzw. wasserlöslichen Substanzen. Bestimmte an sich aus kosmetischer Sicht sehr vorteilhafte Bestandteile der Wasserphase, z.B. Glycerin, aber auch der Ölphase, z.B. Tocopherylacetat wirken sich in höheren Konzentrationen negativ auf die sensorischen Eigenschaften der Zubereitungen aus. Oft äußert sich dies in einem gesteigerten Klebrigkeitsgefühl oder auch Schmierigkeitsgefühl bei der Anwendung entsprechender Zubereitungen, welche dann im Einzelfalle nicht vermarktungsfähig sein können, da sie vom Verbraucher nicht akzeptiert bzw. negativ beurteilt werden.

Es ist zwar bekannt, durch Hinzufügen bestimmter Substanzen, beispielsweise einiger ausgewählter Puderrohstoffe, insbesondere Talkum, dieses Klebrigkeitsgefühl oder auch Schmierigkeitsgefühl zu reduzieren. Davon abgesehen, dass dieses nur selten vollständig gelingt, wird durch einen solchen Zusatz auch die Viskosität des betreffenden Produktes verändert und die Stabilität verringert.

Um die hautbefeuchtende Wirkung von Wasser-in-Öl Emulsionen zu steigern, werden Polyole (Glycerin, Propylenglykol, Butylenglykol, Sorbitol etc.) eingesetzt. Diese Zubereitungen sind besonders mit einem Polyolgehalt von über 5 % überaus klebrig und aus sensorischen Gründen nicht akzeptabel. Nach dem Produktauftrag soll auf der Haut kein oder nur wenig Rückstand verbleiben.

Unter dem Begriff "Viskosität" versteht man die Eigenschaft einer Flüssigkeit, der gegenseitigen laminaren Verschiebung zweier benachbarter Schichten einen Widerstand (Zähigkeit, innere Reibung) entgegenzusetzen. Man

definiert heute diese sogenannte dynamische Viskosität nach $\eta = \tau/D$ als das Verhältnis der Schubspannung zum Geschwindigkeitsgradienten senkrecht zur Strömungsrichtung. Für newtonsche Flüssigkeiten ist $\eta$ bei gegebener Temperatur eine Stoffkonstante mit der SI-Einheit Pascalsekunde (Pa*s).

Der Quotient $v= \eta/\rho$ aus der dynamischen Viskosität $\eta$ und der Dichte $\rho$ der Flüssigkeit wird als kinematische Viskosität v bezeichnet und in der SI-Einheit $m^2/s$ angegeben.

Als Fluidität ($\varphi$) bezeichnet man den Kehrwert der Viskosität ($\varphi=1/\eta$). Bei Salben und dergleichen wird der Gebrauchswert unter anderem mitbestimmt von der sogenannten Zügigkeit. Unter der Zügigkeit einer Salbe oder Salbengrundlage oder dergleichen versteht man deren Eigenschaft, beim Abstechen verschieden lange Fäden zu ziehen; dementsprechend unterscheidet man kurz- und langzügige Stoffe.

Während die graphische Darstellung des Fließverhaltens Newtonscher Flüssigkeiten bei gegebener Temperatur eine Gerade ergibt, zeigen sich bei den sogenannten nichtnewtonschen Flüssigkeiten in Abhängigkeit vom jeweiligen Geschwindigkeitsgefälle D (Schergeschwindigkeit $\gamma$) bzw. der Schubspannung $\tau$ oft erhebliche Abweichungen. In diesen Fällen lässt sich die sogenannte scheinbare Viskosität bestimmen, die zwar nicht der Newton'schen Gleichung gehorcht, aus der sich jedoch durch graphische Verfahren die wahren Viskositätswerte ermitteln lassen.

Die Fallkörperviskosimetrie ist lediglich zur Untersuchung newtonscher Flüssigkeiten sowie von Gasen geeignet. Sie basiert auf dem Stokes-Gesetz, nach dem für das Fallen einer Kugel durch eine sie umströmende Flüssigkeit die dynamische Viskosität $\eta$ aus

$$\eta = \frac{2\, r^2\, (\rho K - \rho Fl)\, g}{9v}$$

bestimmbar ist, wobei r = Radius der Kugel, v = Fallgeschwindigkeit, pK = Dichte der Kugel, $\rho$Fl = Dichte der Flüssigkeit und g = Fallbeschleunigung.

W/O-Emulsionen mit einer geringen Viskosität, die eine Lagerstabilität aufweisen, wie sie für marktgängige Produkte gefordert wird, sind nach dem Stand der Technik nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

W/O-Emulsionen mit einer höheren Viskosität sind durchaus gängige Zubereitungen. Unter Verzicht auf cyclische Silikonöle neigen solche Zubereitungen allerdings dazu, kosmetisch anspruchslos zu wirken und sich durch wenig ansprechendes Hautgefühl auszuzeichnen.

Es hat sich überraschenderweise gezeigt, dass Wasser-in-Öl-Emulsionen mit einem Gehalt an 4-Hydroxyacetophenon den Nachteilen des Standes der Technik abhelfen und gegen physikalische Zersetzung geschützt sind.

Bevorzugte Einsatzkonzentrationen von 4-Hydroxyacetophenon in kosmetischen oder dermatologischen Zubereitungen werden aus dem Bereich von 0,001 % bis 2 %, bevorzugt von 0,01 % bis 1,5 %, insbesondere bevorzugt von 0,1 % bis 1 %, jeweils bezogen auf das Gesamtgewicht der Zubereitungen.

Die kosmetischen und/oder dermatologischen Formulierungen gemäß der Erfindung können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden. Als Grundbestandteile der erfindungsgemäßen Zubereitungen können verwendet werden: Wasser oder wässrige Lösungen; wässrige ethanolische Lösungen; natürliche öle und/oder chemisch modifizierte natürliche öle und/oder synthetische öle;

Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;

Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder-monobutylether, Propylenglykolmonomethyl, -monoethyl- oder -monobutylether, Diet- hylenglykolmonomethyl- oder -monoethylether und analoge Produkte.

Insbesondere werden Gemische der vorstehend genannten Lösungsmittel verwendet.

## SILICONÖLE

Es wird bevorzugt, die Ölphase der erfindungsgemäßen Zubereitungen aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

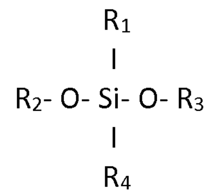

Als erfindungsgemäß vorteilhaft einzusetzende lineare Silicone mit mehreren Siloxyleinheiten werden im Allgemeinen durch Strukturelemente charakterisiert wie folgt:

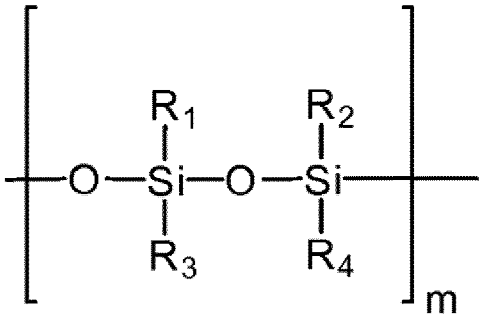

wobei die Siliciumatome mit gleichen oder unterschiedlichen Alkylresten und/oder Arylresten substituiert werden können, welche hier verallgemeinernd durch die Reste Ri - $R_4$ dargestellt sind (will sagen, dass die Anzahl der unterschiedlichen Reste nicht notwendig auf bis zu 4 beschränkt ist), m kann dabei Werte von 2 - 200.000 annehmen.

Erfindungsgemäß vorteilhaft einzusetzende cyclische Silicone werden im Allgemeinen durch Strukturelemente charakterisiert, wie folgt

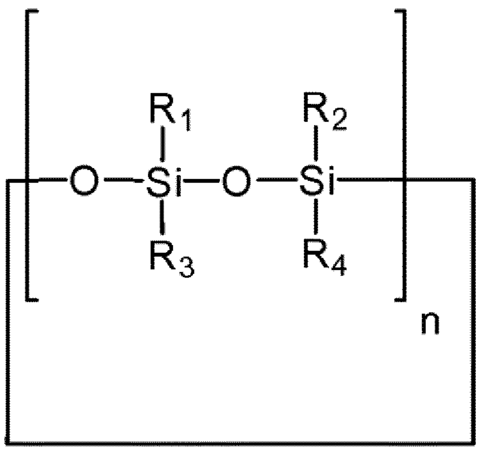

wobei die Siliciumatome mit gleichen oder unterschiedlichen Alkylresten und/oder Arylresten substituiert werden können, welche hier verallgemeinernd durch die Reste $R_1$ bis $R_4$ dargestellt sind (will sagen, dass die Anzahl der unterschiedlichen Reste nicht notwendig auf bis zu 4 beschränkt ist), n kann dabei Werte von 3/2 bis 20 annehmen. Gebrochene Werte für n berücksichtigen, dass ungeradzahlige Anzahlen von Siloxylgruppen im Zyklus vorhanden sein können.

Vorteilhaft wird Phenyltrimethicon als Siliconöl gewählt. Auch andere Silikonöle, beispielsweise Dimethicon (Polydimethylsiloxan), Phenyldimethicon, Cyclomethicon (zum Beispiel Cyclopentasiloxan), Poly(methylphenylsiloxan), Cetyldimethicon, Dimethiconol, Behenoxydimethicon sind vorteilhaft im Sinne der vorliegenden Erfindung zu verwenden.

Die Ölphase der Emulsionen im Sinne der vorliegenden Erfindung besteht erfindungsgemäß vorzugsweise zu 0,5 bis 15 Gew.-% aus Siliconen der unter Punkt (b) aufgeführten Art, wobei es allerdings möglich ist, ohne große Nachteile in Kauf zu nehmen, bis zur Hälfte des Gesamtgewichtes der Ölkomponenten aus der Gruppe anderer Ölkomponenten zu wählen. Diese können dann vorteilhaft gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropy-lmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, sowie synthetische, halbsynthetische und natürliche Gemische solcher Ester, z.B. Jojobaöl.

## WEITERE ÖLKOMPONENTEN

Ferner können die Ölkomponenten der Ölphase vorteilhaft gewählt werden aus der Gruppe der verzweigten und unverzweigten Kohlenwasserstoffe und -wachse, der Dialkylether und/oder Dialkylcarbonate, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen öle, z.B. Olivenöl, Sonnenblumenöl, Sojaöl, Erdnussöl, Rapsöl, Mandelöl, Palmöl, Kokosöl, Palmkernöl und dergleichen mehr.

Erfindungsgemäsß vorteilhaft zu verwendende Fett- und/oder Wachskomponenten können aus der Gruppe der pflanzlichen Wachse, tierischen Wachse, Mineralwachse und petroche- mischen Wachse gewählt werden. Erfindungsgemäß günstig sind beispielsweise Candelilla- wachs, Carnaubawachs, Japanwachs, Reiskeimolwachs, Montanwachs, Jojobawachs, Shea Butter, Bienenwachs, Schellackwachs, Lanolin (Wollwachs), Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise die unter den Handelsbezeichnungen Syncro- wax HRC (Glyceryltribehenat), Syncrowax HGLC (Ci6-36 -Fettsäuretriglycerid) und Syncrowax AW 1 C (C18-C36-Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C30-C50-Alkyl Bienenwachs), Po-lyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C20-C40-Alkylstearat, C20-C40-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett- und/oder Wachskomponenten sowohl einzeln als auch im Gemisch vorliegen.

Auch beliebige Abmischungen solcher öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C12-C15-Alkylbenzoat, Capryl-Caprinsäuretriglycerid, Dicaprylylether.

Besonders vorteilhaft sind Mischungen aus C12-C15-Alkybenzoat und 2-Ethylhexylisostearat, Mischungen aus C12-C15-Alkybenzoat und Isotridecylisononanoat sowie Mischungen aus C12-C15-Alkybenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden.

## SILICONEMULGATOREN

Erfindungsgemäß sind die Siliconemulgatoren aus der Gruppe der Alkyl-Dimethiconcopolyole ausgewählt, insbesondere aus der Gruppe der Verbindungen, welche gekennzeichnet sind durch die folgende chemische Struktur:

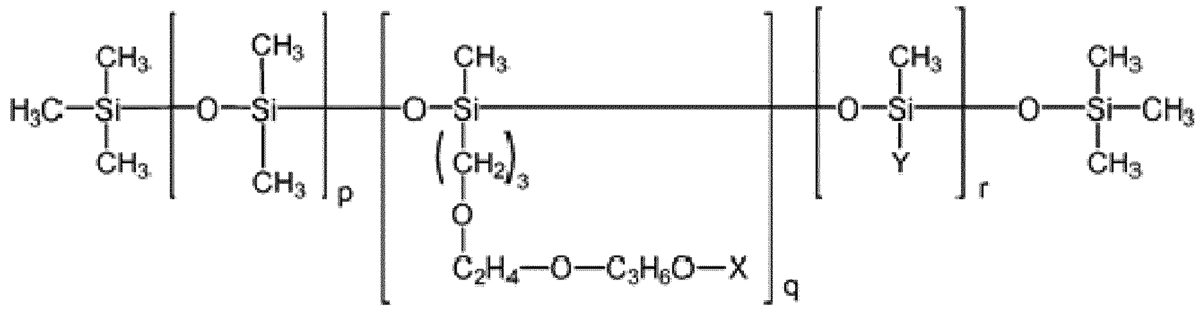

bei welcher X und Y unabhängig voneinander gewählt werden aus der Gruppe H sowie der verzweigten und unverzweigten Alkylgruppen, Acylgruppen und Alkoxygruppen mit 1 bis 24 Kohlenstoffatomen, p eine Zahl von 0 bis 200 darstellt, q eine Zahl von 1 bis 40 darstellt, und r eine Zahl von 1 bis 100 darstellt.

Ein Beispiel für besonders vorteilhaft im Sinne der vorliegenden Erfindung zu verwendende grenzflächenaktiven Substanzen ist das Cetyl PEG/PPG-10/1 Dimethicone (internationaler Name Cetyl Dimethiconcopolyol), welches von der Gesellschaft Evonik unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von der Gesellschaft Evonik unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist. Erfindungsgemäße Emulsionen im Sinne der vorliegenden Erfindung, z.B. in Form einer Hautschutzcreme, einer Hautlotion, einer kosmetischen Milch, beispielsweise in Form einer Sonnenschutzcreme oder einer Sonnenschutzmilch, sind vorteilhaft und enthalten z.B. Fette, öle, Wachse und/oder andere Fettkörper, sowie Wasser und einen oder mehrere Emulgatoren, wie sie üblicherweise für einen solchen Typ der Formulierung verwendet werden.

## WEITERE HILFS- UND ZUSATZSTOFFE

Es ist dem Fachmann natürlich bekannt, dass anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfüm, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und/oder dermatologischen Formulierungen gemäß der Erfindung in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment. Kosmetische Zubereitungen gemäß der Erfindung zum Schutze der Haut vor UV-Strahlen können in verschiedenen Formen vorliegen, wie sie z.B. Üblicherweise für diesen Typ von Zubereitungen eingesetzt werden.

Die kosmetischen Zubereitungen gemäß der Erfindung können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhaft können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.- % beträgt, bezogen auf das Gesamtgewicht der Zubereitung, um

kosmetische Zubereitungen zur Verfügung zu stellen, die die Haut vor dem gesamten Bereich der ultravioletten Strahlung schützen.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

## BEISPIELE

### BEISPIEL 1, VERGLEICHSBEISPIELE V1 und V2

**Messung der Viskosität:**

Verwendetes Gerät: Rheomat 123, proRheo, Althengstett, Deutschland. Als Messkörper wird der Messkörper 1 verwendet. Die Proben wurden bei 25 °C vortemperiert und gemessen. Die Viskosität wird in mPas*s angegeben.

**Experimente:**

Überraschenderweise hat sich gezeigt, dass W/O Emulsionen mit 4-Hydroxyacetophenon als niedermolekulares Additiv einen geringeren Viskositätsabfall aufweisen als niedermolekulare Additive wie zum Beispiel Methylparaben oder Phenoxyethanol. Diese Reduktion der Viskosität bzw. der Konsistenz im Laufe der Lagerung soll vermieden werden, da dies eine Qualitätseinschränkung darstellt. Die folgenden Versuche gemäß Tabelle 1 demonstrieren den Vorteil von 4-Hydroxyacetophenon:

Tabelle 1

Viskosität in mPas*s

| Beispiel | Emulgator | Additiv | Viskosität 1 Tag nach Herstellung | Viskosität 4 Wochen nach Herstellung | Δ in % |
|---|---|---|---|---|---|
| 1 | Cetyl PEG/PPG 10/1 Dimethicone | 4 Hydroxyacetophenon | 16.000 | 15.000 | -6 |
| V1 | Cetyl PEG/PPG 10/1 Dimethicone | Methylparaben | 18.000 | 900 | -50 |
| V2 | Cetyl PEG/PPG 10/1 Dimethicone | ohne | 20.000 | 20.000 | 0 |

Die folgenden Beispielrezepturen 1-3 sollen die Verkörperungen der vorliegenden Erfindungen verdeutlichen. Die Angaben beziehen sich stets auf Gewichts-%, sofern nicht andere Angaben gemacht werden.

| W/O EMULSIONEN | |
|---|---|
| **Beispielrezeptur 1** | **Menge** |
| Cetyl PEG/PPG-10/1 Dimethicon (Abil® EM 90) | 4,0 |
| Dimethicon + Trimethylsiloxysilicat | 3,5 |
| Caprylsäure / Caprinsäure Triglycerid | 5,0 |
| Dicaprylylether | 5,0 |
| Squalan | 0,5 |
| Dicaprylylcarbonat | 3,0 |
| Dimethicon | 10,0 |
| Disteardimonium Hectorit | 1,0 |
| Propylencarbonat | q.s. |
| Lauroyl Lysin | 2,5 |

(fortgesetzt)

| W/O EMULSIONEN | |
|---|---|
| **Beispielrezeptur 1** | **Menge** |
| Natriumchlorid | 2,0 |
| 4-Hydroxyacetophenon | 0,5 |
| Glycerin | 7,0 |
| Zitronensäure (pH 4,5 eingestellt) | q.s. |
| Wasser 100 % | Ad 100 |
| **Beispielrezeptur 2** | **Menge** |
| Cetyl PEG/PPG-10/1 Dimethicon (Abil® EM 90) | 4,0 |
| Dimethicon + Trimethylsiloxysilicat | 3,5 |
| Caprylsäure / Caprinsäure Triglycerid | 5,0 |
| Dicaprylylether | 5,0 |
| Squalan | 0,5 |
| Dicaprylylcarbonat | 3,0 |
| Dimethicon | 10,0 |
| Disteardimonium Hectorit | 1,0 |
| Propylencarbonat | q.s. |
| Lauroyl Lysin | 2,5 |
| Natriumchlorid | 2,0 |
| Methylparaben | 0,5 |
| Glycerin | 7,0 |
| Zitronensäure (pH 4,5 eingestellt) | q.s. |
| Wasser 100 % | Ad 100 |
| **Beispielrezeptur 3** | **Menge** |
| Cetyl PEG/PPG-10/1 Dimethicon (Abil® EM 90) | 4,0 |
| Dimethicone + Trimethylsiloxysilicate | 3,5 |
| Caprylsäure / Caprinsäure Triglycerid | 5,0 |
| Dicaprylylether | 5,0 |
| Squalan | 0,5 |
| Dicaprylylcarbonat | 3,0 |
| Dimethicon | 10,0 |
| Disteardimonium Hectorit | 1,0 |
| Propylencarbonat | q.s. |
| Lauroyl Lysin | 2,5 |
| Natriumchlorid | 2,0 |
| Glycerin | 7,0 |
| Methylisothiazolinon | 0,02 |
| Zitronensäure (pH 4,5 eingestellt) | q.s. |

| | |
|---|---|
| Wasser 100 % | Ad 100 |

(fortgesetzt)

| **Beispielrezeptur 4** | **Menge** |
|---|---|
| PEG-40 Sorbitan Perisostearat | 2,0 |
| Polyglyceryl-3 Diisostearat | 1,0 |
| Paraffinum Liquidum | 15,0 |
| Cera Microcristallina+Paraffinum Liquidum | 3,0 |
| Parfuem | 0,3 |
| Magnesiumsulfat | 0,7 |
| EDTA | 0,2 |
| Glycerin | 5,0 |
| 4-Hydroxyacetophenon | 0,5 |
| Natriumcitrat gelöst in Wasser (pH 5 eingestellt) | q.s. |
| Zitronensäure (pH 5 eingestellt) | 0,9 |
| Wasser 100 % | Ad 100 |
| **Beispielrezeptur 5** | **Menge** |
| PEG-40 Sorbitan Perisostearat | 2,0 |
| Polyglyceryl-3 Diisostearat | 1,0 |
| Paraffinum Liquidum | 15,0 |
| Cera Microcristallina+Paraffinum Liquidum | 3,0 |
| Parfüm | 0,3 |
| Magnesiumsulfat | 0,7 |
| EDTA | 0,2 |
| Glycerin | 5,0 |
| Natriumcitrat gelöst in Wasser (pH 5 eingestellt) | q.s. |
| Zitronensäure | 0,9 |
| Wasser 100 % | Ad 100 |
| **Beispielrezeptur 6** | **Gew.-%** |
| PEG-40 Sorbitan Perisostearat | 2,0 |
| Polyglyceryl-3 Diisostearat | 1,0 |
| Paraffinum Liquidum | 15,0 |
| Cera Microcristallina+Paraffinum Liquidum | 3,0 |
| Parfüm | 0,3 |
| Magnesiumsulfat | 0,7 |
| EDTA | 0,2 |
| Glycerin | 5,0 |
| Phenoxyethanol | 0,5 |
| Natriumcitrat gelöst in Wasser (pH 5 eingestellt) | q.s. |
| Zitronensäure | 0,9 |
| Wasser 100 % | Ad 100 |

(fortgesetzt)

| Beispielrezeptur 7 | Menge |
|---|---|
| PEG-40 Sorbitan Perisostearat | 2,0 |
| Polyglyceryl-3 Diisostearat | 1,0 |
| Paraffinum Liquidum | 15,0 |
| Cera Microcristallina+Paraffinum Liquidum | 3,0 |
| Parfüm | 0,3 |
| Magnesiumsulfat | 0,7 |
| EDTA | 0,2 |
| Glycerin | 5,0 |
| Methylparaben | 0,5 |
| Natriumcitrat gelöst in Wasser (pH 5 eingestellt) | q.s. |
| Zitronensäure | 0,9 |
| Wasser 100 % | Ad 100 |
| **W/O FOUNDATIONS** | |
| **Beispielrezeptur 8** | **Menge** |
| Cetyl PEG/PPG-10/1 Dimethicon (Abil® EM 90 | 4,0 |
| Dimethicon | 10,0 |
| Dicaprylylcarbonat | 5,0 |
| Dicaprylylether | 5,0 |
| Caprylsäure / Caprinsäure Triglycerid | 2,0 |
| Butylmethoxydibenzoylmethan | 0,8 |
| Octocrylen | 3,0 |
| Disteardimonium Hectorit | 1,0 |
| Propylencarbonat | q.s. |
| Titandioxid | 1,0 |
| Talcum | 1,0 |
| Farbpigmente (IC 77891, CI 77492, CI 77491, CI 77499, CI 77007) | 6,0 |
| Natriumchlorid | 2,0 |
| 4-Hydroxyacetophenon | 0,5 |
| Glycerin | 5,0 |
| Zitronensäure (pH 4,5 eingestellt) | q.s. |
| Wasser 100 % | Ad 100 |
| **Beispielrezeptur 9** | **Menge** |
| PEG-10 Dimethicone | 3,0 |
| Bis-PEG/PPG-14/14 Dimethicone | 1,5 |
| Cyclomethicone | 5,0 |
| Dimethicone | 3,0 |
| Dicaprylylether | 3,0 |
| Butylmethoxydibenzoylmethan | 0,8 |

(fortgesetzt)

| Beispielrezeptur 9 | Menge |
|---|---|
| Octylmethoxyzimtsäureester | 4,0 |
| Octocrylen | 1,0 |
| Disteardimonium Hectorit | 1,0 |
| Propylencarbonat | q.s. |
| Titandioxid | 1,0 |
| Talcum | 1,0 |
| Farbpigmente (IC 77891, CI 77492, CI 77491, CI 77499, CI 77007) | 6,0 |
| Magnesiumsulfat | 0,9 |
| Ethanol | 1,0 |
| 4-Hydroxyacetophenon | 0,5 |
| Butylenglycol | 5,0 |
| Wasser 100 % | Ad 100 |
| **Beispielrezeptur 10** | **Menge** |
| Mineralöl | 20,0 |
| Cera Microcristallina+Paraffinum Liquidum | 5,0 |
| Lanolinalkohol | 3,0 |
| Parfüm | 0,3 |
| Magnesiumsulfat | 0,5 |
| Magnesium Stearat | 0,1 |
| Aluminium Stearat | 0,2 |
| Glycerin | 5,0 |
| 4-Hydroxyacetophenon | 0,3 |
| Decyloleat | 0,9 |
| Octyldodecanol | 0,3 |
| Zitronensäure (pH 4 eingestellt) | q.s. |
| Wasser 100 % | Ad 100 |

## Patentansprüche

**1.** W/O-Emulsionen enthaltend

(a) mindestens einen Silikonemulgator vom Typ der Alkyl-Dimethicon-Copolyole und
(b) 4-Hydroxyacetophenon.

**2.** W/O-Emulsionen nach Anspruch 1, **dadurch gekennzeichnet, dass** sie 4-Hydroxyacetophenon (Komponente b) in Mengen von 0,001 bis 2 Gew.-% - bezogen auf die Emulsionen - enthalten.

**3.** W/O-Emulsionen den Ansprüchen 1 und/oder 2, **dadurch gekennzeichnet, dass** sie 4-Hydroxyacetophenon (Komponente b) in Mengen von 0,1 bis 1 Gew.-% - bezogen auf die Emulsionen - enthalten.

**4.** W/O-Emulsionen nach mindestens einem der Ansprüche 1 bis 3, **dadurch gekennzeichnet, dass** sie als Hautschutzcreme, Reinigungsmilch, Sonnenschutzlotion, Nährcreme, Tagecreme oder Nachtcreme vorliegen.

## Claims

1. W/O emulsions comprising

   (a) at least one silicon emulsifier of the alkyl-dimethicone-copolyol type and
   (b) 4-hydroxyacetophenone.

2. W/O emulsions according to Claim 1, **characterized in that** they comprise said 4-hydroxyacetophenone (component b) in amounts of from 0.001 to 2 % by weight - calculated on said emulsions.

3. W/O emulsions according to Claims 1 and/or 2, **characterized in that** they comprise said 4-hydroxyacetophenone (component b) in amounts of from 0.1 to 1 % by weight - calculated on said emulsions.

4. W/O emulsions according to any of Claims 1 to 3, **characterized in that** they represent skin care crèmes, cleaning milks, sun care lotions, nutrient crèmes, day crèmes or night crèmes.

## Revendications

1. Emulsions eau-dans-huile contenant

   (a) au moins un émulsionnant siliconé du type des alkyl-diméthicone-copolyols et
   (b) de la 4-hydroxyacétophénone.

2. Emulsions eau-dans-huile selon la revendication 1, **caractérisées en ce qu'**elles contiennent de la 4-hydroxyacétophénone (composant b) en des quantités de 0,001 à 2% en poids par rapport aux émulsions.

3. Emulsions eau-dans-huile selon la revendication 1 et/ou 2, **caractérisées en ce qu'**elles contiennent de la 4-hydroxyacétophénone (composant b) en des quantités de 0,1 à 1% en poids par rapport aux émulsions.

4. Emulsions eau-dans-huile selon au moins une des revendications 1 à 3, **caractérisées en ce qu'**elles sont présentes sous forme de crème de protection de la peau, lait nettoyant, lotion de protection solaire, crème nourrissante, crème de jour ou crème de nuit.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2003070200 A1